# EUROPÄISCHE PATENTSCHRIFT

(11) **EP 1 358 053 B1**
(45) Veröffentlichungstag und Bekanntmachung des Hinweises auf die Patenterteilung: **25.04.2007**
(21) Anmeldenummer: 02718071.0
(22) Anmeldetag: 31.01.2002
(51) Int. Cl.: B29B 13/08, B29C 35/08, A61L 27/16

(54) **VERFAHREN ZUR HERSTELLUNG VON IMPLANTATTEILEN AUS HOCHVERNETZTEM UHMWPE**
METHOD FOR PRODUCING IMPLANT PARTS FROM HIGHLY CROSS-LINKED UHMWPE
PROCEDE DE REALISATION DE PARTIES D'IMPLANT EN UHMWPE HAUTEMENT RETICULE

(30) Priorität: 05.02.2001 DE 10105085
(43) Veröffentlichungstag der Anmeldung: 05.11.2003
(73) Patentinhaber: Plus Orthopedics AG, 6343 Rotkreuz (CH)
(72) Erfinder: SCHMOTZER, Hans, CH-8048 Zürich-Altstetten (CH)
(74) Vertreter: Popp, Eugen
(86) Internationale Anmeldenummer: PCT/EP2002/001011
(87) Internationale Veröffentlichungsnummer: WO 2002/062548

(56) Entgegenhaltungen:
- EP-A- 0 737 481
- EP-A- 1 072 274
- US-A- 6 165 220
- US-B1- 6 174 934

## Beschreibung

Die vorliegende Erfindung betrifft ein Verfahren zur Herstellung von Implantatteilen aus hochvernetztem Polyethylen, im folgenden als UHMWPE bezeichnet. Häufig werden sie hierbei als Beschichtung und/oder Gleitlagermaterial eingesetzt, um die physikomechanischen Eigenschaften zu optimieren.

In der Vergangenheit wurde hierzu "normales" ultrahochmolekulargewichtiges Polyethylen eingesetzt, das jedoch ungewünschte physiologisch relevante Nebenwirkungen aufwies, die auf eine unzureichende Homogenität und Abriebsfestigkeit des verwendeten UHMWPE zurückzuführen sind. So entstehen beispielsweise beim Einsatz von "normalem" UHMWPE als Gleitlager in künstlichen Hüftgelenken durch Abnutzung mikroskopisch kleine Teilchen, die sich im umliegenden Gewebe verteilen und zu Gewebsnekrose und Osteolyse führen können. Der Körper reagiert mit Entzündungen und Gewebsveränderungen. Somit ist ein - an sich sehr wünschenswerter - Langzeiteinsatz derartiger Implantatteile nicht möglich. Wiederkehrende Operationen sind nötig.

In der Folgezeit wurde deshalb für derartige Implantatteile hochvernetztes Polyethylen eingesetzt, das durch zusätzliche Vernetzungsschritte aus herkömmlichem UHMWPE hergestellt wurde. Diese Vernetzung wurde üblicherweise über radikalische Zwischenstufen erreicht, wobei die Radikale entweder mittels ionisierender Strahlung oder, alternativ hierzu, chemisch erzeugt wurden.

Eine wesentliche Voraussetzung für den Einsatz von derartig hergestelltem hochvernetztem Polyethylen in einem Organismus ist allerdings, daß die Radikale lediglich einen übergangszustand darstellen und im fertigen Produkt, d.h., vor der Verwendung als Implantat vollständig abgefangen sind, da sie sonst, wie allgemein bekannt, unspezifisch, beispielsweise als Cancerogene gewebszerstörend wirken können.

Die vollständige Beseitigung der Radikale erweist sich jedoch als problematisch, da die Stabilität und somit die Lebensdauer von Radikalen durch unterschiedliche kinetische und/oder thermodynamische Effekte "positiv" beeinflußt wird. So ist in der vorliegenden Matrix, nämlich vernetztem Polyethylen, einerseits sowohl eine stabilisierende Wirkung der Alkylgruppen als auch eine sterische Hinderung durch sperrige Substituenten, respektive starke Vernetzung, bzw. eine mangelhafte Beweglichkeit, die durch die starke Vernetzung bedingt ist, gegeben, so daß manche Radikale kaum Gelegenheit zum Rekombinieren finden und deshalb mehr oder weniger isoliert in der Matrix verbleiben, wobei jedoch Folgereaktionen und eine Diffusion der Radikale prinzipiell möglich sind.

Letztendlich führt eine unvollständige Beseitigung der Radikale demzufolge zu einer Verschlechterung der Eigenschaften des Implantatteils, beispielsweise durch eine nachfolgende Oxidation, und infolgedessen zu den vorgenannten Problemen hinsichtlich der Homogenität und der Abriebfestigkeit des eingesetzten Polyethylens.

Dieses Problem ist seit längerem bekannt und wurde auf vielfältige Weise zu lösen versucht.

Im wesentlichen beruhen diese Ansätze darauf, das Werkstück bei erhöhter Temperatur im Bereich seines Schmelzpunkts zu tempern, um den in dem Werkstück verbliebenen Radikalen auf diese Weise eine erhöhte Mobilität zu verschaffen und eine Rekombination zu ermöglichen.

Diese Vorgehensweise wird beispielsweise in der US-A 6,017,975 beschrieben, die davon ausgeht, die bestrahlte Matrix solange auf eine Temperatur von etwa 150°C zu erwärmen, bis im wesentlichen alle freien Radikale rekombiniert sind. Außer Betracht bleibt hierbei jedoch, daß ein reines Erwärmen der Matrix, die aufgrund ihrer hohen Vernetzung allenfalls zähflüssig wird, nicht die sterische Hinderung, die durch die Vernetzung bedingt ist, beseitigen kann, so daß die Mobilität der Radikale zwar etwas erhöht ist, eine vollständige Abreaktion der Radikale, insbesondere der sterisch gehinderten, jedoch in einer vernünftigen Zeitspanne praktisch nicht möglich ist.

Im Ergebnis bedeutet dies, daß die Zeit zur Rekombination aller freien Radikale, insbesondere auch wegen der von Fall zu Fall nicht konstanten Struktur der Matrix, nicht vorhersagbar ist und nahezu beliebig lang sein kann.

Einen ähnlichen Weg beschreiten die US-A 5,414,049 und EP 0 722 973 A1. Dort wird die bestrahlte Matrix ebenfalls getempert, wobei die Zielsetzung der US-Schrift ebenfalls in Richtung der Beseitigung freier Radikale geht, während dies in der EP-Schrift eine Begleitreaktion ist und mittels des Temperns eigentlich eine mögliche spätere Schrumpfung der Matrix vorweggenommen werden soll. Die vorgenannten Probleme eines reinen Temperns treffen auch bezüglich der beiden letztgenannten Druckschriften zu.

Einen völlig anderen Weg beschreitet die US-A 5,577,368, die versucht mittels eines äußeren Wasserstoffpartialdruckes während des Bestrahlens die Anzahl verbleibender freier Radikale zu reduzieren. Nachteilig ist hierbei jedoch, daß die Vernetzung des Polyethylens gegenüber den vorgenannten Verfahren vermindert ist, da Kettenbindungsbrüche mit Wasserstoff abgesättigt werden können ohne daß sich eine Kohlenstoff-Kohlenstoff-Bindung ausbildet.

Die WO 97/29793 A1 beschreibt Prothesen aus bestrahltem UHMWPE und andere Gegenstände aus diesem Material, jeweils ohne freie Radikale, sowie mehrere Verfahren zur Herstellung von solchem UHMWPE, wobei die Bestrahlung durchweg mit Elektronenstrahlen durchgeführt wird.

Die EP 1 072 274 A1 beschreibt ein Verfahren zur Herstellung von Gleitlagerschichten, indem ein polymeres Material, vorzugsweise UHMWPE einer Bestrahlung mit Gamma-Strahlen ausgesetzt wird, um ein gewünschtes Ausmaß an Vernetzung zu bewirken. Dieses Material wird anschließend oberhalb der Schmelztemperatur vernetzt, um die Widerstandsfähigkeit gegen oxidativen Abbau zu erhöhen. Anschließend wird das Produkt in die gewünschte Form gebracht und erneut mit Gamma-Strahlen bestrahlt, um eine Sterilisierung zu bewirken.

Aus der EP 1 072 275 ist ein Verfahren zur Herstellung von Gleitlagerschichten aus UHMWPE für orthopädische Implantate bekannt, bei dem eine Vernetzung des Polymeren durch Bestrahlen mit Gamma-Strahlen, Röntgenstrahlen, Ultraviolett-Strahlen, Neutronenstrahlen, Protonenstrahlen oder Elektronenstrahlen erfolgt, um mindestens einen Teil des UHMWPE zu vernetzen.

Aus der EP 1 086 709 A1 ist schließlich ein Verfahren zur Verbesserung der Gleitlagereigenschaften von Formteilen aus UHMWPE für orthopädische Implantate bekannt, in dem das UHMWPE mit Elektronenstrahlen bestrahlt und so eine Verminderung bzw. Beseitigung der freien Radikale und Vernetzung des Produktes erreicht werden.

Bei keinem der vorgenannten Verfahren wird eine Nachbehandlung in der Weise durchgeführt, daß im wesentlichen nicht rekombinierte freie Radikale mittels Mikrowellenstrahlung und/oder Ultraschall angeregt werden.

Die Aufgabe der vorliegenden Erfindung ist demgemäß, ein Verfahren zur Herstellung von Implantatteilen zur Verfügung zu stellen, in denen das UHMWPE zum einen hochvernetzt ist und in dem darüber hinaus der Radikalgehalt gegenüber dem Stand der Technik reduziert ist. Eine weitere Aufgabe der Erfindung besteht darin, diese Ziele mittels einer Nachbehandlung in einer möglichst schonenden Weise zu erreichen, indem hohe Verfahrenstemperaturen vermieden werden. Durch die Erfindung kann also eine ähnliche Vernetzungsdichte wie bei den bisher bekannten Produkten und eine Verminderung der freien Radikale erreicht werden, und zwar bei deutlich niedrigeren Verfahrenstemperaturen.

Diese Aufgabe wird durch ein Verfahren zur Herstellung von Implantatteilen aus hochvernetztem Polyethylen gelöst, das folgende Schritte aufweist:
- Bestrahlen eines Rohlings aus ultrahochmolekulargewichtigem Polyethylen mit ionisierender Strahlung, insbesondere Röntgen-, Gamma oder Elektronenstrahlung, zur Erzeugung von Radikalen,
- Nachbehandeln des bestrahlten Rohlings,
- Anpassen der Form des nachbehandelten Rohlings zur Erzeugung eines Implantteils,
wobei das Nachbehandeln dadurch erfolgt, daß nicht-rekombinierte freie Radikale mittels Mikrowellenstrahlung und/oder Ultraschall angeregt werden, um eine im wesentlichen vollständige Rekombination der freien Radikale zu gewährleisten. Dadurch wird auch die Vernetzung des UHMWPE weiter optimiert. Die so hergestellten Produkte finden Verwendung als Implantatteile in der humanmedizinischen Anwendung.

Der wesentliche Kern der Erfindung liegt darin, daß Moleküle und/oder Molekülteile mit einer polaren Struktur mittels Mikrowellenstrahlung anregbar sind. Die Anregung erfolgt hierbei im unmittelbaren Umfeld des polaren Strukturelements, beispielsweise des Makromoleküls, und führt mittelbar zu einer Erwärmung der polaren Komponente. Eine Erwärmung der weiteren Umgebung des polaren Strukturelements durch die Mikrowellenstrahlung tritt nicht ein und ist allenfalls auf Wärmeleitung innerhalb der Matrix zurückzuführen. Somit ist es mittels Mikrowellenstrahlung möglich, die Erwärmung innerhalb der Matrix auf die unmittelbare Umgebung der Radikale zu beschränken.

Die Erhöhung der kinetischen Energie der freien Radikale sowie deren Umgebung wiederum führt zu einer erhöhten Beweglichkeit der beteiligten Elektronen und daraus resultierend zu einer Wanderung des radikalischen Elektrons, beispielsweise durch Isomerisierung, bis ein geeigneter Reaktionspartner, beispielsweise ein weiteres radikalisches Elektron in dessen Reichweite gelangt und die beiden Elektronen unter Ausbildung einer neuen σ-Bindung rekombinieren.

Bei der Anwendung der Mikrowellenstrahlung zur Förderung der Rekombination von freien Radikalen ist es prinzipiell möglich, mit der Bestrahlung der Matrix, welche die Radikale enthält, bei Raumtemperatur zu beginnen, wobei eine Temperaturerhöhung stattfindet. Es ist jedoch ebenso möglich, die Matrix zusätzlich unterstützend auf konventionelle Art zu erwärmen. Beispielsweise kann hierbei eine aus der vorangegangenen Behandlung mit ionisierender Strahlung resultierende Restwärme genutzt werden, sofern der Rohling vor der Bestrahlung erwärmt wurde. Eine erneute Erwärmung ist selbstverständlich ebenso möglich.

Der wesentliche Effekt der Erfindung, nämlich die gegenüber der Umgebung zusätzliche Temperaturerhöhung im Umfeld von polaren Strukturen in der Matrix bleibt von einer konventionellen Erwärmung im wesentlichen unbeeinflußt; es wird lediglich eine durch die Temperaturerhöhung bedingte Erhöhung der Diffusions-, respektive Wanderungsgeschwindigkeit der radikalischen Elektronen bewirkt.

Sofern von einer konventionellen vorherigen Erwärmung der Matrix abgesehen wird, kann die Temperatur des Rohlings bzw. der den Rohling bildenden Matrix auch nur mittels Mikrowellenstrahlung soweit erhöht werden, daß eine akzeptable Reaktionsgeschwindigkeit erreicht wird. Dies ist beispielsweise dann der Fall, wenn die Energiedichte der auf den Rohling angewandten Mikrowellenstrahlung im Bereich von 10 mW/cm² bis 10 W/cm² und bevorzugt im Bereich von 100 mW/cm² bis 5 W/cm² liegt. Hierdurch ist es möglich, im Rohling eine Temperatur über 60°C, z.B. bis zu seinem Schmelzpunkt, vorzugsweise im Bereich von 80°C bis 140°C und besonders bevorzugt bis 130°C zu erreichen.

Die Frequenzen der auf den Rohling angewandten Mikrowellenstrahlung liegen im allgemeinen im Bereich zwischen 20 MHz und 300 GHz, wobei die jeweiligen Frequenzen insbesondere darauf abgestimmt sind, Kohlenstoff-Kohlenstoff- und Kohlenstoff-Wasserstoff-Bindungen im Umfeld der Radikale anzuregen.

Erfindungsgemäß ist es möglich, das Nachbehandeln des bestrahlten Rohlings lediglich mit Mikrowellenstrahlung durchzuführen. Ferner kann unterstützend oder anstelle von Mikrowellenstrahlung Ultraschall eingesetzt werden. Hierbei wird der Effekt ausgenützt, daß Ultraschall sehr hochfrequente mechanische Erschütterungen bewirkt, die das Innere des beschallten Körpers erwärmen. Der Rohling kann hierbei entweder als ganzes bestrahlt und somit erwärmt werden; es ist jedoch aufgrund der guten Fokussierbarkeit von Ultraschallwellen ebenso möglich, diese gebündelt auf bestimmte ausgewählte Bereiche des Rohlings einwirken zu lassen, beispielsweise in Form einer Abrasterung.

Bei der Anwendung von Ultraschall als Energieträger wird vorzugsweise eine Schallstärke im Bereich von 0,2 W/cm² bis 20 W/cm² angewandt, die ausreichend ist, um den Rohling bis zum oder sogar über seinen Schmelzpunkt zu erwärmen. Hierdurch wird wiederum die Diffusionsgeschwindigkeit der freien Radikale erhöht. Bevorzugt liegt die Schallstärke im Bereich von 1 W/cm² bis 15 W/cm², und besonders bevorzugt im Bereich von 5 W/cm² bis 10 W/cm². Desweiteren sind in der flüssigen Phase der Matrix sonochemische Reaktionen möglich, die auf Kavitationen zurückgehen. Bei diesen Kavitationen treten kurzzeitig extrem hohe Temperaturen auf, die zur Moleküldissoziation und einer nachfolgenden Rekombination der entstandenen Radikale führen, wodurch die Wanderungsfähigkeit des ursprünglichen radikalischen Elektrons begünstigt wird.

Die Anwendung einer konventionellen, nicht auf die Anwendung von Ultraschall zurückgehenden Erwärmung ist unterstützend ebenso möglich. Auch hier kann die Restwärme aus dem vorangegangenen Bestrahlungsschritt genutzt werden.

Bei einer Kombination von Mikrowellenstrahlung mit einer Ultrabeschallung ergänzen sich die oben erläuterten positiven Wirkungen der beiden Energieträgerformen gegenseitig.

Die Bestrahlung des Rohlings und die erfindungsgemäße Nachbehandlung finden mit einer Strahlrichtung statt, die im wesentlichen parallel zur kleinsten Ausdehnung des Rohlings, d.i. der Dicke ist, wobei die Dicke des Rohlings im Bereich von 0,5 cm bis 10 cm, bevorzugt im Bereich von 1 cm bis 7 cm und besonders bevorzugt nicht über 4 cm liegt. Durch die Wahl dieser Strahlrichtung wird eine maximale Durchdringung erreicht.

Als ionisierende Strahlung wird Elektronenstrahlung, alternativ oder ergänzend hierzu auch Gammastrahlung und/oder Röntgenstrahlung eingesetzt. Dabei ist Elektronenstrahlung bevorzugt, da deren zur Radikalbildung benötigte Einwirkzeit kürzer ist und infolgedessen die Möglichkeit einer Oxidation - sofern die Bestrahlung nicht unter Sauerstoffausschluß stattfindet - vermindert ist. Darüber hinaus kann über eine Variation der Energie der Elektronen die Eindringtiefe der Elektronen variiert werden, so daß der Grad der Vernetzung und der Kristallinität des UHMWPE-Produkts beeinflußt werden kann. Die angewandte Energie liegt hierbei im allgemeinen im Bereich von 0,5 MeV bis 15 MeV, bevorzugt im Bereich von 5 MeV bis 12 MeV und besonders bevorzugt im Bereich von 8 MeV bis 11 MeV, z.B. bei etwa 10 MeV.

Gammastrahlung wird bevorzugt dann eingesetzt, wenn hohe Eindringtiefen erforderlich sind. Die angewandte Dosis liegt hierbei im allgemeinen im Bereich von 2,5 Mrad bis 25 Mrad, vorzugsweise von 4 Mrad bis 16 Mrad , und besonders bevorzugt von 8 Mrad bis 12 Mrad, z.B. bei etwa 10 Mrad.

Auch die Bestrahlung des Rohlings mit ionisierenden Strahlen wird im allgemeinen bei einer Temperatur bis zu seinem Schmelzpunkt, insbesondere im Bereich von 60°C bis 160°C, vorzugsweise im Bereich von 80°C bis 140°C und besonders bevorzugt im Bereich von 110°C bis 130°C durchgeführt. Gegenüber einer Bestrahlung bei Raumtemperatur, die ebenfalls möglich ist, wirkt sich die erhöhte Temperatur dahingehend vorteilhaft aus, daß der amorphe Anteil der Rohlingmatrix erhöht wird. Dies hat zur Folge, daß die Kristallinität und somit auch die Rißbildungsgefahr des Rohlings sinkt. Darüber hinaus wird die Beweglichkeit der einzelnen Molekülketten erhöht und es ist bei Bestrahlung eine gute Quervernetzung möglich. Zudem steigt die Reaktionsgeschwindigkeit der Vernetzung mit der Temperatur.

Gemäß einer weiteren Ausführungsform der Erfindung wird der Rohling während und/oder nach der Bestrahlung mit den ionisierenden Strahlen während einer Temperphase für eine Dauer bis zu einem Tag, vorzugsweise bis zu etwa 12 Stunden und besonders bevorzugt für einen Zeitraum von 2 Stunden bis 4 Stunden bei einer Temperatur in den oben genannten Temperaturbereichen getempert.

Der hierin begründete Vorteil liegt darin, daß freie, thermodynamisch und kinetisch nicht gehinderte, Radikale, die noch nicht abreagiert, bzw. zu einer σ-Bindung rekombiniert haben, während der Temperphase weitgehend abreagieren können.

Gemäß einer besonders vorteilhaften Ausführungsform des Verfahrens wird der bestrahlte Rohling während der Temperphase mit Mikrowellenstrahlung und/oder Ultraschallbehandlung nachbehandelt, wobei zur Unterstützung der Wirkungen eine erhöhte, insbesondere die während der Temperphase aufrechterhaltene, Temperatur verwendet wird.

Nach der Behandlung mit Mikrowellen und/oder Ultraschall und der Temperphase wird der Rohling gemäß einem vordefinierten Abkühlprogramm auf Raumtemperatur abgekühlt, wobei darauf zu achten ist, daß er spannungsfrei bleibt. Dies ist erfindungsgemäß dadurch möglich, daß das Abkühlen mit einer Abkühlrate im Bereich von 1°C bis 10°C pro Stunde, bevorzugt mit 2,5°C bis 7°C pro Stunde und besonders bevorzugt mit im wesentlichen 5°C pro Stunde erfolgt.

Auf diese Weise ist es, insbesondere in Kombination mit der vorhergehenden Temperphase möglich, ein späteres Schrumpfen des Rohlings zu vermeiden.

Gemäß einer weiteren Ausführungsform des erfindungsgemäßen Verfahrens werden zumindest Teilschritte des Verfahrens unter gegenüber Atmosphärendruck reduziertem Druck, beispielsweise im Vakuum, z.B. dem der Wasserstrahlpumpe oder im Hochvakuum durchgeführt. Dies hat den Vorteil, daß Reaktionen der Rohlingmatrix mit dem umgebenden Gas minimiert bzw. vermieden werden. Besonders vorteilhaft wirkt sich hierbei der Ausschluß bzw. die Minimierung von Oxidation aus, was zu einer Versprödung und zu einer Alterung des Fertigprodukts führen würde.

Ferner ist es möglich, zumindest Teilschritte des Verfahrens unter einer sauerstoff- und/oder feuchtigkeitsreduzierten Atmosphäre und/oder unter Inertgas durchzuführen.

Als Teilschritte des Verfahrens sind in beiden Fällen insbesondere die Bestrahlungs-, die Temper-, die Abkühl- und die Nachbehandlungsphase zu verstehen.

Anstelle eines gegenüber Atmosphärendruck reduzierten Drucks ist zur Vermeidung von Oxidation auch der Ausschluß oder zumindest die Reduktion des Gehaltes an Sauerstoff und/oder Feuchtigkeit in der den Rohling umgebenden Atmosphäre möglich. Auch der vollständige Ersatz von atmosphärischer Luft mit einem reaktionsträgen Gas oder mit Inertgas ist möglich. Geeignet sind hierfür beispielsweise Stickstoff oder die Edelgase, insbesondere Argon.

Gemäß einer weiteren vorteilhaften Ausführungsform der Erfindung wird das erfindungsgemäße Nachbehandeln in einer mit Wasserstoff angereicherten Atmosphäre durchgeführt. Der hieraus resultierende Vorteil liegt in der.hohen Diffusionsfähigkeit von Wasserstoff. Der.Wasserstoff kann hierbei in die Polymermatrix eindiffundieren und mit freien radikalischen Elektronen unter Ausbildung einer Kohlenstoff-WasserstoffBindung reagieren. Darüberhinaus ist Wasserstoff geeignet, eine reduzierende Atmosphäre zur Verfügung zu stellen, die einer eventuellen Oxidation entgegenwirkt.

Vorteilhaft erfolgt die Anpassung der Form des nachbehandelten Rohlings in spanabhebender Weise.

Ein hochvernetztes Polyethylen, das nach dem vorliegenden Verfahren behandelt ist, wird als Implantatteil verwendet.

Der hierin begründete Vorteil liegt darin, daß ein derartiges hochvernetztes Polyethylen besonders abriebfest ist und praktisch keine freien Radikale enthält. Ein derartig hergestelltes Implantatteil ist deshalb gegenüber herkömmlich bekannten Implantatteilen physiologisch unbedenklich und weist eine gegenüber bekannten Implantatteilen stark erhöhte Biokompatibilität und somit Lebensdauer auf.

Nachfolgend wird die Erfindung anhand eines Ausführungsbeispiels beschrieben.

### Beispiel:

Ein handelsübliches Halbzeug aus UHMWPE, beispielsweise GUR 1020 (spezifisches Gewicht: 927-944 g/l, Molekulargewicht: 3-4 Mio. g/mol, kein Stabilisator) gemäß ISO 5834-2 wird als Rohling mit einer Dicke von 40 mm in einem auf 125°C vorgewärmten Ofen für 3 Stunden auf 125°C erwärmt. Danach wird der Rohling mit einer Strahlrichtung, die parallel.zu seiner geringsten Ausdehnung verläuft, mit Elektronen einer Energie von 10 MeV bestrahlt. Die Gesamtleistung beträgt 120 kW. Anschließend wird der Rohling unter Beibehaltung der Temperatur mit Mikrowellen einer Energie von 2 W/cm² und Ultraschall einer Stärke von ebenfalls 2 W/cm² für 30 Minuten bestrahlt und anschließend mit einer konstanten Rate von 5°C pro Stunde auf Raumtemperatur abgekühlt. Die Weiterbearbeitüng des Rohlings erfolgt mittels spanabhebender Formgebung dergestalt, daß ein Gleitlager für eine Hüftprothese erzeugt wird, gegen das der Kopf eines Hüftprothesenschafts artikuliert.

## Patentansprüche

1. Verfahren zur Herstellung von Implantatteilen aus hochvernetztem Polyethylen mittels folgender Schritte:
- Bestrahlen eines Rohlings aus ultrahochmolekulargewichtigem Polyethylen mit ionisierender Strahlung, insbesondere Röntgen-, Gamma- oder Elektronenstrahlung, zur Erzeugung von Radikalen in dem Rohling,
- Nachbehandeln des bestrahlten Rohlings,
- Anpassung der Form des nachbehandelten Rohlings zur Erzeugung eines Implantatteils,
**dadurch gekennzeichnet, daß**
das Nachbehandeln **dadurch** erfolgt, daß nicht rekombinierte freie Radikale mittels Mikrowellenstrahlung und/oder Ultraschall angeregt werden, um eine im wesentlichen vollständige Rekombination der freien Radikale zu gewährleisten.

2. Verfahren nach Anspruch 1,
**dadurch gekennzeichnet, daß**
die Frequenzen der auf den Rohling angewandten Mikrowellenstrahlung im Bereich zwischen 20 MHz und 300 GHz liegen, wobei die angewandten Frequenzen insbesondere darauf abgestimmt sind, Kohlenstoff-Kohlenstoff- und Kohlenstoff-Wasserstoffbindungen im Umfeld der Radikale anzuregen.

3. Verfahren nach einem oder mehreren der vorhergehenden Ansprüche,
**dadurch gekennzeichnet, daß**
die auf den Rohling angewandte Energiedichte der Mikrowellenstrahlung im Bereich von 10 mW/cm² bis 10 W/cm² und bevorzugt im Bereich von 100 mW/cm² bis 5 W/cm² liegt, wobei der Rohling zum Nachbehandeln mittels Mikrowellenstrahlung und/oder Ultraschall auf eine Temperatur über 60°C, vorzugsweise von 80°C bis 140°C und besonders bevorzugt bis 130°C erwärmt wird.

4. Verfahren nach einem oder mehreren der vorhergehenden Ansprüche,
**dadurch gekennzeichnet, daß**
die Schallstärke des Ultraschalls im Bereich von 0,2 W/cm² bis 20 W/cm², bevorzugt im Bereich von 1 W/cm² bis 15 W/cm², und besonders bevorzugt im Bereich von 5 W/cm² bis 10 W/cm² liegt.

5. Verfahren nach einem oder mehreren der vorhergehenden Ansprüche,
**dadurch gekennzeichnet, daß**
die Bestrahlung im wesentlichen parallel zur kleinsten Ausdehnung des Rohlings erfolgt, wobei die Dicke des Rohlings im Bereich von 0,5 cm bis 10 cm, bevorzugt im Bereich von 1 cm bis 7 cm und besonders bevorzugt nicht über 4 cm liegt.

6. Verfahren nach einem oder mehreren der vorhergehenden Ansprüche,
**dadurch gekennzeichnet, daß**
der Rohling vor oder bei der Bestrahlung mit den ionisierenden Strahlen auf eine Temperatur im Bereich von 60°C bis 160°C, vorzugsweise im Bereich von 80°C bis 140°C und besonders bevorzugt im Bereich von 110°C bis 130°C erwärmt wird, wobei der Rohling vorzugsweise während und gegebenenfalls nach der Bestrahlung und Nachbehandlung im wesentlichen auf der Erwärmungstemperatur gehalten wird, wobei er nach der Bestrahlung während einer Dauer von bis zu 1 Tag, vorzugsweise bis zu etwa 12 Stunden und besonders bevorzugt für einen Zeitraum von 2 Stunden bis 4 Stunden bei einer Temperatur in den vorgenannten Temperaturbereichen getempert und danach während einer Abkühlphase gemäß einem vordefinierten Abkühlprogramm auf Raumtemperatur abgekühlt wird, wobei die Abkühlrate zweckmäßig im Bereich von 1°C bis 10°C pro Stunde, bevorzugt im Bereich von 2,5°C bis 7°C pro Stunde liegt.

7. Verfahren nach einem oder mehreren der vorhergehenden Ansprüche,
**dadurch gekennzeichnet, daß**
das Nachbehandeln des bestrahlten Rohlings während der Temperphase oder während dessen Abkühlung erfolgt.

8. Verfahren nach einem oder mehreren der vorhergehenden Ansprüche,
**dadurch gekennzeichnet, daß**
zumindest Teilschritte des Verfahrens, insbesondere die Bestrahlungs-, die Temper-, die Abkühl- und die Nachbehandlungsphase des Rohlings unter gegenüber Atmosphärendruck reduziertem Druck, vorzugsweise im Vakuum erfolgen.

9. Verfahren nach einem oder mehreren der vorhergehenden Ansprüche,
**dadurch gekennzeichnet, daß**
zumindest Teilschritte des Verfahrens, insbesondere die Bestrahlungs-, die Temper-, die Abkühl- und die Nachbehandlungsphase des Rohlings unter einer sauerstoff- und/oder feuchtigkeitsreduzierten Atmosphäre und/oder unter Inertgas erfolgen, und daß das Nachbehandeln auch in einer mit Wasserstoff angereicherten Atmosphäre erfolgen kann.

10. Verfahren nach einem oder mehreren der vorhergehenden Ansprüche,
**dadurch gekennzeichnet, daß**
die Anpassung der Form des nachbehandelten Rohlings durch Spanabhebung erfolgt.

11. Verfahren nach einem oder mehreren der vorhergehenden Ansprüche,
**dadurch gekennzeichnet, daß**
zur Erzeugung von Radikalen angewandte Elektronenstrahlung in einem Energiebereich von 0,5 MeV bis 15 MeV, bevorzugt im Bereich von 5 MeV bis 12 MeV und besonders bevorzugt im Bereich von 8 MeV bis 11 MeV liegt, und daß die Dosis von zur Erzeugung von Radikalen angewandter Gammastrahlung in einem Bereich von 2,5 Mrad bis 25 Mrad, vorzugsweise in einem Bereich von 4 Mrad bis 16 Mrad und besonders bevorzugt in einem Bereich von 8 Mrad bis 12 Mrad liegt.

## Claims

1. Method of producing implant elements from highly cross-linked polyethylene by means of the following steps:
- irradiation of a blank of ultra-high molecular weight polyethylene with ionising radiation, especially X-radiation, gamma radiation or electron radiation, to generate free radicals in the blank,
- after-treatment of the irradiated blank,
- adaptation of the shape of the after-treated blank to produce an implant element,
**characterised in that**
the after-treatment is carried out by the excitation of non-recombined free radicals by means of microwave radiation and/or ultrasound to ensure substantially complete recombination of the free radicals.

2. Method according to claim 1,
**characterised in that**
the frequency of the microwave radiation applied to the blank is in the range between 20 MHz and 300 GHz, the frequencies applied being adapted especially to excite carbon-carbon and carbon-hydrogen bonding in the field surrounding the free radicals.

3. Method according to one or more of the preceding claims,
**characterised in that**
the energy density of the microwave radiation applied to the blank is in the range from 10 mW/cm² to 10 W/cm² and preferably in the range from 100 mW/cm² to 5 W/cm², the blank being heated, for the after-treatment, to a temperature of above 60°C, preferably from 80°C up to 140°C and especially preferably up to 130°C, by means of microwave radiation and/or ultrasound.

4. Method according to one or more of the preceding claims,
**characterised in that**
the sound intensity of the ultrasound is in the range from 0.2 W/cm² to 20 W/cm², preferably in the range from 1 W/cm² to 15 W/cm², and especially preferably in the range from 5 W/cm² to 10 W/cm².

5. Method according to one or more of the preceding claims,
**characterised in that**
the irradiation is carried out substantially parallel to the smallest dimension of the blank, the thickness of the blank being in the range from 0.5 cm to 10 cm, preferably in the range from 1 cm to 7 cm and especially preferably being not more than 4 cm.

6. Method according to one or more of the preceding claims,
**characterised in that**
prior to or during irradiation with the ionising rays, the blank is heated to a temperature in the range from 60°C to 160°C, preferably in the range from 80°C to 140°C and especially preferably in the range from 110°C to 130°C, the blank being maintained substantially at the heating temperature preferably during, and optionally after, the irradiation and after-treatment, wherein, after the irradiation, it is tempered for a period of up to 1 day, preferably up to about 12 hours and especially preferably for a period of from 2 hours to 4 hours, at a temperature in the above-mentioned temperature ranges, and after that, during a cooling phase, it is cooled to room temperature in accordance with a predefined cooling programme, the cooling rate advantageously being in the range from 1°C to 10°C per hour, preferably in the range from 2.5°C to 7°C per hour.

7. Method according to one or more of the preceding claims,
**characterised in that**
the after-treatment of the irradiated blank is carried out during the tempering phase or during the cooling phase thereof.

8. Method according to one or more of the preceding claims,
**characterised in that**
at least a subset of method steps, especially the irradiation, the tempering, the cooling and the after-treatment phases of the blank, are carried out at reduced pressure relative to atmospheric pressure, preferably *in vacuo.*

9. Method according to one or more of the preceding claims,
**characterised in that**
at least a subset of method steps, especially the irradiation, the tempering, the cooling and the after-treatment phases of the blank, are carried out under a reduced-oxygen and/or reduced-humidity atmosphere and/or under inert gas, and **in that** the after-treatment can also be carried out in a hydrogen-enriched atmosphere.

10. Method according to one or more of the preceding claims,
**characterised in that**
the adaptation of the shape of the after-treated blank is carried out by material removal.

11. Method according to one or more of the preceding claims,
**characterised in that**
electron radiation applied to generate free radicals is in an energy range from 0.5 MeV to 15 MeV, preferably in the range from 5 MeV to 12 MeV and especially preferably in the range from 8 MeV to 11 MeV, and the dose of gamma radiation applied to generate free radicals is in a range from 2.5 Mrad to 25 Mrad, preferably in a range from 4 Mrad to 16 Mrad and especially preferably in a range from 8 Mrad to 12 Mrad.

## Revendications

1. Procédé de fabrication de parties d'implant en polyéthylène hautement réticulé au moyen des étapes suivantes :
- Irradiation d'une ébauche en polyéthylène de masse moléculaire ultra élevée au moyen d'un rayonnement ionisant, en particulier des rayons X, des rayons gamma ou un faisceau d'électrons, afin de générer des radicaux dans l'ébauche,
- Retraitement de l'ébauche irradiée,
- Adaptation de la forme de l'ébauche retraitée pour produire une partie d'implant,
**caractérisé en ce que** le retraitement se fait en excitant les radicaux libres non recombinés au moyen d'un rayonnement hyperfréquence et/ou au moyen d'ultrasons afin de garantir une recombinaison sensiblement complète des radicaux libres.

2. Procédé selon la revendication 1, **caractérisé en ce que** les fréquences du rayonnement hyperfréquence qui est appliqué à l'ébauche se situent dans la plage de 20 MHz à 300 GHz, les fréquences appliquées étant notamment accordées pour exciter des liaisons carbone-carbone et carbone-hydrogène dans l'environnement des radicaux.

3. Procédé selon l'une ou plusieurs des revendications précédentes, **caractérisé en ce que** la densité d'énergie du rayonnement hyperfréquence appliqué à l'ébauche se situe dans la plage de 10 mW/cm² à 10 W/cm², de préférence dans la plage de 100 mW/cm² à 5 W/cm², l'ébauche étant chauffée à une température supérieure à 60°C, de préférence dans la plage de 80°C à 140°C ou, de façon particulièrement préférée, à 130°C, pour le retraitement par un rayonnement hyperfréquence et/ou des ultrasons.

4. Procédé selon l'une ou plusieurs des revendications précédentes, **caractérisé en ce que** l'intensité acoustique des ultrasons se situe dans la plage de 0,2 W/cm² à 20 W/cm², de préférence dans la plage de 1 W/cm² à 15 W/cm² et de façon particulièrement préférée dans la plage de 5 W/cm² à 10 W/cm².

5. Procédé selon l'une ou plusieurs des revendications précédentes, **caractérisé en ce que** l'irradiation se fait sensiblement parallèlement à la plus petite dimension de l'ébauche, l'épaisseur de l'ébauche se situant dans la plage de 0,5 cm à 10 cm, de préférence dans la plage de 1 cm à 7 cm et, de façon particulièrement préférée, n'étant pas supérieure à 4 cm.

6. Procédé selon l'une ou plusieurs des revendications précédentes, **caractérisé en ce que**, avant ou pendant l'irradiation avec les rayons ionisants, l'ébauche est chauffée à une température dans la plage de 60°C à 160°C, de préférence dans la plage de 80°C à 140°C et de façon particulièrement préférée dans la plage de 110°C à 130°C, l'ébauche étant de préférence maintenue, pendant et, le cas échéant, après l'irradiation et le retraitement, sensiblement à la température de chauffage et étant, après l'irradiation, étuvée pendant une durée allant jusqu'à 1 jour, de préférence jusqu'à environ 12 heures et de façon particulièrement préférée pendant une durée allant de 2 heures à 4 heures à une température comprise dans les plages de température précitées, et ensuite refroidie à la température ambiante pendant une phase de refroidissement suivant un programme de refroidissement prédéfini, la vitesse de refroidissement se situant judicieusement dans la plage de 1°C à 10°C par heure, de préférence dans la plage de 2,5°C à 7°C par heure.

7. Procédé selon l'une ou plusieurs des revendications précédentes, **caractérisé en ce que** le retraitement de l'ébauche irradiée se fait pendant la phase d'étuvage ou pendant son refroidissement.

8. Procédé selon l'une ou plusieurs des revendications précédentes, **caractérisé en ce qu'**au moins des étapes partielles du procédé, en particulier les phases d'irradiation, d'étuvage, de refroidissement et de retraitement de l'ébauche, se font sous une pression réduite par rapport à la pression atmosphérique, de préférence sous vide.

9. Procédé selon l'une ou plusieurs des revendications précédentes, **caractérisé en ce qu'**au moins des étapes partielles du procédé, en particulier les phases d'irradiation, d'étuvage, de refroidissement et de retraitement de l'ébauche, se font sous une atmosphère appauvrie en oxygène et/ou en humidité et/ou sous un gaz inerte et **en ce que** le retraitement peut également se faire sous une atmosphère enrichie en hydrogène.

10. Procédé selon l'une ou plusieurs des revendications précédentes, **caractérisé en ce que** l'adaptation de la forme de l'ébauche retraitée se fait par enlèvement de matière.

11. Procédé selon l'une ou plusieurs des revendications précédentes, **caractérisé en ce que** le rayonnement électronique utilisé pour produire des radicaux se situe dans une plage d'énergie de 0,5 MeV à 15 MeV, de préférence dans la plage de 5 MeV à 12 MeV et de façon particulièrement préférée dans la plage de 8 MeV à 11 MeV, et **en ce que** la dose de rayonnement gamma utilisée pour produire des radicaux se situe dans une plage de 2,5 Mrad à 25 Mrad, de préférence dans une plage de 4 Mrad à 16 Mrad et de façon particulièrement préférée dans une plage de 8 Mrad à 12 Mrad.
